(19) Europäisches Patentamt
European Patent Office
Office européen des brevets

(11) **EP 3 831 304 B1**

(12) **EUROPEAN PATENT SPECIFICATION**

(45) Date of publication and mention
of the grant of the patent:
**23.08.2023 Bulletin 2023/34**

(21) Application number: **20212256.0**

(22) Date of filing: **07.12.2020**

(51) International Patent Classification (IPC):
***A61B 5/363*** (2021.01)      ***A61B 5/35*** (2021.01)

(52) Cooperative Patent Classification (CPC):
**A61B 5/363; A61B 5/35;** A61B 5/287

(54) **INTRA-CARDIAC PATTERN MATCHING**

INTRAKARDIALER MUSTERABGLEICH

APPARIEMENT DE MOTIF INTRACARDIAQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **06.12.2019 US 201962944557 P
19.11.2020 US 202016952943**

(43) Date of publication of application:
**09.06.2021 Bulletin 2021/23**

(73) Proprietor: **Biosense Webster (Israel) Ltd.
Yokneam 2066717 (IL)**

(72) Inventors:
• **YARNITSKY, Jonathan
2066717 Yokneam (IL)**

• **NAKAR, Elad
2066717 Yokneam (IL)**
• **BAR-TAL, Meir
2066717 Yokneam (IL)**
• **RUBINSTEIN, Vladimir
2066717 Yokneam (IL)**
• **BEN-DOR, Amir
2066717 Yokneam (IL)**
• **TURGEMAN, Aharon
2066717 Yokneam (IL)**

(74) Representative: **Carpmaels & Ransford LLP
One Southampton Row
London WC1B 5HA (GB)**

(56) References cited:
US-A1- 2017 042 443      US-A1- 2018 008 203
US-A1- 2018 042 510      US-A1- 2018 199 847

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

**FIELD OF INVENTION**

The present application generally relates to intra-cardiac (IC) signals, and specifically to detecting IC signals having similar morphologies.

**BACKGROUND**

For correctly mapping regions of a heart chamber which generate an arrhythmia, it is essential that only signals, or beats, exhibiting that specific arrhythmia are captured. Signals from effects such as ectopic beats, mechanical stimulation of the tissue, and changes in arrythmia morphology due to alternative activation patterns with the same cycle length, should be ignored. Introducing signals generated by such effects into a map of the heart chamber will cause inaccuracies in the local activation map, and the deformed visualization of the arrhythmia will make it difficult for a medical professional to clearly identify the arrhythmia mechanisms.

US2018/0008203 A1 discloses automatic creation of multiple electroanatomic maps. Cardiac electrograms are recorded in a plurality of channels. Beats are classified automatically into respective classifications according to a resemblance of the morphologic characteristics of the beats to members of a set of templates. Respective electroanatomic maps of the heart are generated from the classified beats.

US2017/0042443 A1 discloses matching and tracking time sequences of heart activation. A method includes receiving a first group of electrocardiograph (ECG) signals derived from a single heartbeat and generated at a respective plurality of electrodes on a catheter in a heart of a subject, formulating a template relating times of annotations of the first group of the ECG signals, and assigning the template an index. The method further includes receiving a second group of ECG signals derived from a subsequent single heartbeat and generated at the electrodes, calculating times of annotations of the second group, formulating a comparison between the template and the times of annotations of the second group, and, when the comparison indicates that the times of annotations of the second group correspond to the template, assigning the index to the second group of ECG signals, and presenting graphically on a display an occurrence of the template relative to a timeline representing heartbeats from the heart.

US2018/0042510 A1 discloses identifying signals having the same morphology. A method includes selecting an initial set of electrocardiograph (ECG) signals taken over a single heartbeat of a human subject, the set having respective morphologies to be used as a template for an arrhythmia of the subject, and receiving a subsequent set of ECG signals taken over a subsequent heartbeat of the human subject. The method also includes performing a cross-correlation between the initial set and the subsequent set, so as to generate a correlation coefficient that is a measure of a goodness of fit between geometries of the initial set and the subsequent set, and, when the correlation coefficient exceeds a threshold coefficient, accepting the subsequent heartbeat as having been caused by the arrhythmia.

US2018/0199847 A1 discloses a system and method for generating premature ventricular contraction electrophysiology maps. A method of mapping arrhythmic activity, such as premature ventricular contraction ("PVC") activity, using an electroanatomical mapping system includes defining at least two arrhythmia template signals. Electrophysiology data points, each including an electrophysiological signal, are collected. A morphological similarity between the electrophysiological signal and a first arrhythmia template signal is computed; if this exceeds a preset threshold, then the electrophysiology data point is added to a corresponding arrhythmia map. If it does not, a morphological similarity between the electrophysiological signal and a second arrhythmia template signal is computed. If this exceeds the preset threshold, then the electrophysiology data point is added to a corresponding arrhythmia map. If neither exceeds the preset threshold, then the electrophysiology data point can be used to establish an additional arrhythmia map by defining an additional arrhythmia template signal.

**SUMMARY**

Methods, apparatuses, and systems for medical procedures and signal mapping regions of the heart chamber are disclosed herein. Under one example, unipolar pattern intra-cardiac (IC) electrogram (EGM) signals are received from an area of a heart. The IC EGM signals are received from a plurality of activity channels corresponding to a plurality of electrodes of one or more catheters. A pattern of interest (POI) of the IC EGM signals is selected to encompass at least a portion of a cycle length of the IC EGM signals corresponding to a cardiac condition, such as a problematic cardiac activity, e.g., an arrhythmia activation. A template POI is generated. The template POI is created from a plurality of template channels based on the IC EGM signals within a window of interest WOI. Subsequent electrical activity comprising IC EGM signals is received from the plurality of activity channels corresponding to the plurality of electrodes of the one or more catheters. Weights are applied to the plurality of activity channels to generate weighted subsequent electrical activity. The weights are based on a derivative of a pattern within each template channel. A correlation score is calculated by comparing the weighted subsequent electrical activity to the template POI. A determination is then made regarding whether that correlation score is above a threshold correlation value. A subsequent electrical activity beat that has a maximum correlation value that is greater than the threshold correlation value may be determined to be problematic electrical activity that is similar to the problematic electrical activity identified in the POI of the template pattern.

**BRIEF DESCRIPTION OF THE DRAWINGS**

A more detailed understanding can be had from the following description, given by way of example in conjunction with the accompanying drawings wherein:

FIG. 1 is a diagram of an exemplary system in which one or more features of the present invention may be implemented;

FIG. 2 is a flowchart for determining a correlation between a template and subsequent electrical activity;

FIG. 3 is a chart of a finite impulse response (FIR); and,

FIG. 4 is a schematic diagram illustrating operation blocks of a phase shift operation, according to an embodiment.

**DETAILED DESCRIPTION**

Cardiac electrical signals can be categorized into different groups. A first group may include sinus rhythms which are exhibited when the heart rate is within its normal range. A second group may include different types of arrhythmias or irregular heartbeats, which can occur when the electrical impulses that coordinate the heart rhythm do not travel normally. There are two major types of arrhythmia: tachycardia, where the heart beats too fast, and bradycardia, where the heart beats too slow. The third group of cardiac electrical signals may be mechanically induced resulting from a catheter being maneuvered within a chamber of the heart of a patient undergoing a cardiac procedure. Often, the beats that are of clinical relevance are the arrhythmias. Thus, it is a goal of the present invention to provide a tool to assist the physician in focusing in on these clinically relevant heartbeats, while filtering out those heartbeats, e.g., sinus rhythms and mechanically induced beats, that are not of clinical interest. One way to identify electrical signals representative of an arrhythmia is to compare a current group of electrical signals to electrical signals that previously have been identified as problematic arrhythmias. However, conventional filtering modules that are currently available suffer a drawback in that they may only allow differentiation of atrial activations based upon cycle length. Multiple activation sources (focal or cyclic) may have similar cycle lengths and, thus, may only be distinguishable based upon their unique sequence of activation defined by their wave propagation. Such differences may not be captured utilizing such existing conventional filtering modules.

The techniques disclosed herein enable discrimination between atrial activations as captured on IC reference signals, based on their unique morphology and sequence of activation, using correlation techniques disclosed herein. Activations of similar cycle length can be identified, along with unwanted mechanically induced beats caused by catheter tissue irritation, and other causes. Additionally, changes to IC signals can be frequently caused by movement of the reference catheter leading to unstable reference and potentially inaccurate data acquisition. The techniques disclosed herein incorporate sensitivity to such changes and can prevent acquisition when movement of the reference catheter occurs.

Notably, the techniques provided herein allow for automated differentiation of IC activations based on the similarity of an activation sequence to a previously determined template sequence. The techniques provided herein allow for sensitivity for both clinical and mechanical changes (e.g., reference catheter movement) which may not be easily identified by a user.

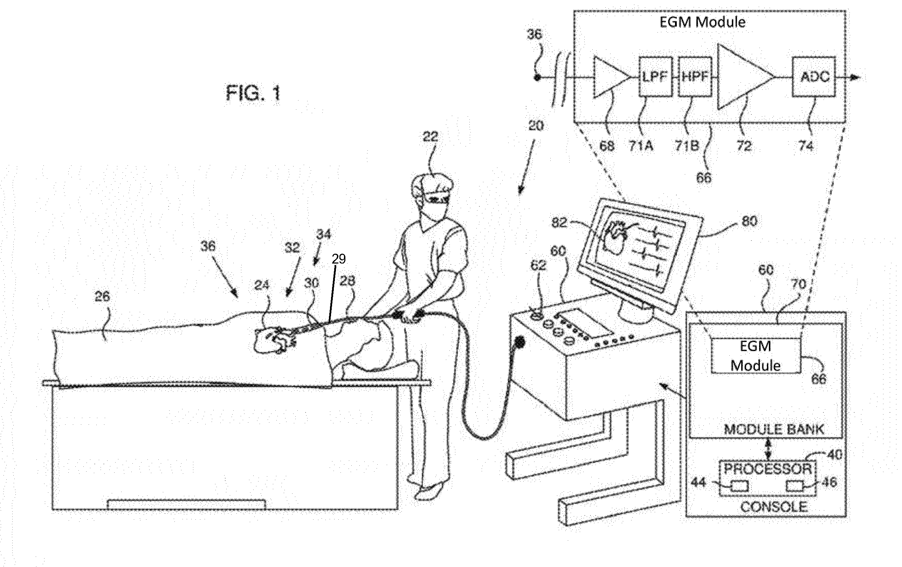

According to exemplary embodiments, unipolar intra-cardiac (IC) signals from within a chamber in the heart are recorded using a catheter inserted into the heart. The unipolar signals may mitigate or eliminate the effects of local activation changes, which may not reflect an actual change in chamber activation. Further, the unipolar signals may exhibit greater temporal stability in comparison to bipolar signals. A selection of a pattern of interest (POI) is received within a window of interest WOI. The WOI captures the entire cycle length for a single heartbeat and collects all of the data relating to that specific activation. Additional WOIs are utilized for capturing the entire cycle length of subsequent heartbeats and collecting all data relating to those heartbeats. The POI captures a specific segment within the WOI of the heartbeat that is of clinical interest, such as where the arrhythmia activation is exhibited. The selection of the POI may be made based on cardiac activity that a medical professional may identify as an arrhythmia activation. Alternatively, the selection of the POI may be performed automatically by a processor (e.g., processor 40 of FIG. 1) which forms a portion of the present invention. The arrhythmia activity may be identified by observing a representation of the arrhythmia (e.g., IC EGM signals) on a monitor or other display. The POI associated with the arrhythmia may be captured and stored and may include the signal or group of signals that correspond to the arrhythmia. The problematic cardiac activity identified via the POI may be used to generate a template for the given problematic cardiac activity such that subsequent cardiac activity that correlates to the problematic cardiac activity may be identified, as disclosed herein.

Subsequent cardiac activity (e.g., subsequent cardiac beats) is compared to the template created based on the identified problematic cardiac activity to determine if the subsequent cardiac activity is correlated to the template. As disclosed herein, the correlation is determined based on a comparison of the multichannel unipolar template cardiac signals and the multichannel unipolar subsequent cardiac signals (e.g., via a Pearson correlation calculation). A weighted correlation score is assigned to the comparison of the multi-channel cardiac signals based on the multichannel unipolar signals to determine if the subsequent cardiac activity correlates to the template. One or more filters may be applied in order to remove baseline wander, which is a low-frequency artefact in the cardiac signal caused by movement by respiration and other movement of the patient. Based on the techniques disclosed herein, subsequent cardiac activity that correlates to the template is indicated to the medical professional. Such activity may be indicated on an image or rendering of the heart such that areas of the heart that exhibit such correlating activity are emphasized (e.g., via color, pattern, marking, etc.) via a display or other visual medium.

FIG. 1 illustrates an exemplary system for performing an invasive medical procedure including an apparatus 20 in accordance with an exemplary embodiment of the present invention. The medical procedure is to be performed by a medical professional 22, and, as an example, the medical procedure provided herein is assumed to include acquisition of intra-cardiac (IC) electrogram (EGM) signals from a heart 24 of a human patient 26. Generally, an IC EGM signal is a biomedical signal that measures electrical currents generated in muscles (e.g., cardiac tissue) during activity, such as contraction.

In order to acquire the IC EGM signals, the medical professional 22 may insert a probe 28 into a sheath 30 that has been pre-positioned within a lumen, e.g., an artery or a vein, of the human patient 26. The sheath 30 is positioned so that a distal end 32 of the probe 28 may enter the heart 24 of the patient 26, after exiting a distal end 34 of the sheath 30, and contact tissue of the heart 24.

The probe 28 may comprise any type of catheter 29 that can be inserted into the heart 24 of the patient 26, and that can be tracked, typically using a magnetic tracking system and/or an impedance measuring system. For example, probe 28 may comprise a lasso catheter, a shaft-like catheter, or a PentaRay™ catheter, produced by Biosense Webster® of Diamond Bar, CA, or catheters generally similar to these catheters. Biosense Webster also produces a magnetic tracking system and an impedance measuring system that may be used in embodiments of the present invention.

The probe 28 includes one or more electrodes 36 and/or one or more catheters 29 in combination with the respective one or more electrodes 36, which are used to acquire the IC EGM signals used by a processor 40, included in the apparatus 20, in performing the techniques described herein. The processor 40, in addition to acting as a central processing unit, may include real-time noise reduction circuitry 44, typically configured as a field programmable gate array (FPGA), followed by an analog-to-digital (A/D) signal conversion integrated circuit 46. The processor 40 can pass the signal from the A/D circuit 46 to another processor and can be programmed to perform the algorithms disclosed herein.

The processor 40 is located in an operating console 60 of the apparatus 20. The operating console 60 comprises controls 62 which may be used by the medical professional 22 to communicate with the processor 40. During the procedure, the processor 40 communicates with an EGM module 66 in a module bank 70 in order to acquire IC EGM signals as well as to perform the algorithms disclosed herein.

The EGM module 66 receives IC EGM signals from the electrode 36. In one embodiment, the IC EGM signals are transferred, in the EGM module 66, through a low noise pre-amplifier 68, through one or more low pass filters 71A, and through one or more high pass filters 71B, to a main amplifier 72. The EGM module 66 also comprises an analog to digital converter (ADC) 74, which transfers digitized values of the IC EGM signals to the processor 40 for implementation by the processor 40 of the algorithms described herein. Such components of the EGM module are standard and are provided to clean up the IC EGM signals by removing noise and compensating for interference and interruptions. Typically, the processor 40 controls the operation of the pre-amplifier 68, the low and high pass filters 71A and 71B, the amplifier 72, and the ADC 74.

For simplicity, FIG. 1 illustrates the EGM module 66 as having one channel for receiving signals from the electrode 36. However, it should be understood that the EGM module 66 typically comprises multiple channels substantially similar to that shown. For example, the EGM module 66 may comprise ten or more channels corresponding to the relative electrical activity sensed by ten different electrodes 36.

The EGM module 66 enables the processor 40 to acquire and analyze electrical signals received by the electrode 36, including the IC EGM signals referred to herein. The IC EGM signals are typically presented to the medical professional 22 as voltage-time graphs, which are updated in real time, on a display screen 80.

The software for the processor 40 and for the module bank 70 may be downloaded to the processor 40 in electronic form, over a network, for example. Alternatively, or additionally, the software for the processor 40 and for the module bank 70 may be provided on non-transitory tangible media, such as optical, magnetic, or electronic storage media.

In order to operate the apparatus 20, the module bank 70 typically comprises modules in addition to the EGM module 66 described above. For example, the module bank 70 may include one or more tracking modules (not shown) enabling the processor 40 to track the distal end of the probe 28. For simplicity, such other modules are not illustrated in FIG. 1. All modules utilized to operate the apparatus 20 may comprise hardware as well as software elements.

In addition to the display screen 80 being utilized for presenting the IC EGM signals acquired by the electrodes 36, the display screen 80 may also be utilized for presenting the results of the techniques described herein. For example, the results of the techniques described herein may be incorporated into a map 82 of the heart 24 displayed on the display screen 80.

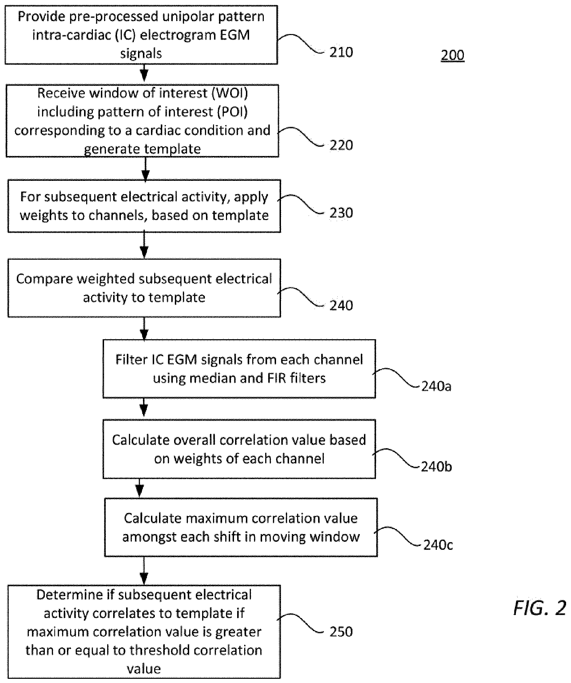

Referring now to FIG. 2, there is shown therein the steps of a process 200 for determining whether subsequent electrical activity correlates to a previously generated template for a given problematic cardiac activity, and whether the value of that correlation exceeds a threshold correlation value.

At step 210 of process 200 of FIG. 2, the catheter 29 may be inserted into a patient's heart 24 and may be configured to detect IC EGM signals at a plurality of points of the heart 24. The IC EGM signals may be sensed by electrodes 36 that are part of the catheter 29. The IC EGM signals may be unipolar IC signals. The signals may be provided on a display, such as display 80 of FIG. 1. As shown at step 210, unipolar pattern IC EGM signals from a point on a heart 24 may be provided to a user (e.g., the medical professional 22 as shown in Fig. 1) or to the processor 40 (Fig. 1). The unipolar pattern IC EGM signals may include a pattern corresponding to a cardiac condition, such as mapped arrhythmia, within a given window of time.

According to an exemplary embodiment, the unipolar pattern IC EGM signals provided at step 210 may be pre-processed such that one or more filters may be applied to the IC EGM signals prior to the template creation. It will be understood that the pre-processing techniques disclosed in step 240a may be applied to the IC EGM signals at step 210 prior to template creation. Such filters may include median and FIR filters to be described in more detail below. Such median and FIR filters are conventional and well known in the art and are utilized for removing baseline wander caused by a patient's respiration and movements often occurring during the medical procedures described herein.

At step 220, the medical professional 22 or the processor 40 may define a WOI that includes a pattern of interest (POI) corresponding to the cardiac condition, such as the problematic cardiac activity, e.g., the arrhythmia. For example, the segment of time on the ECG/EGM signal stream where the activation pattern is observed. As the electrodes 36 of the catheter 29 detect IC EGM signals at a plurality of points within the heart 24, those IC EGM signals are presented to the apparatus 20 as a stream of IC EGM signals or data. In known ways, that stream of IC EGM signals may be divided into sections or segments corresponding to a single contraction or beat of the heart, which is referred to as the WOI.

The POI is a segment of time on the ECG/EGM signals stream where the activation pattern is observed and captured on the reference catheter. Correlation will only be calculated for the pattern selected by the POI. The medical professional 22 or processor 40 may provide reference annotations that indicate the onset and offset of the activation corresponding to the observed problematic cardiac activity, e.g., the arrhythmia. The POI may be defined based on these annotations. Further, a POI may be extracted from the pattern exhibited by the multi-channel unipolar IC EGM signals contained within the WOI. A template pattern may be generated based on the POI such that the template pattern may be the extracted POI or may be a filtered or otherwise adjusted version of the POI. The POI may then be used for a correlation comparison, as described further below. The WOI may include IC EGM signals provided by multiple channels each corresponding to signals received by respective electrodes of the catheter 29. The multiple IC EGM signals may exhibit a pattern that the template may be based upon. When capturing a pattern, a WOI (e.g., a WOI that spans 62.5 seconds) may be saved in a memory accessible by the processor (e.g., processor 40).

It should be noted that the IC EGM signals captured by the electrodes 36 may capture activity of both the atria and the ventricle. When generating a template at step 220 of process 200, the last activation in the identified WOI can be an overlapping atrial and ventricle pattern. However, such an overlapping pattern may not be optimized for mapping. An advanced reference annotation may be applied as the reference criteria for mapping and such overlapping activations may be identified so that the pattern within the WOI is set around the last reference annotation which is representative only of an atrial activation. An advanced reference annotation-based algorithm may use a center of energy approach to one or more reference calculations, which may provide greater stability, especially for beats of different morphology. Methods and algorithms for analyzing multi-channel electro-cardiogram signals generated during a medical procedure and determining a reference annotation time are disclosed in US Patent No. 9,259,165 entitled Determination of Reference Annotation Time from Multi-Channel Electro-Cardiogram signals (Rubenstein, et al.). In addition, such an algorithm may differentiate between atrial and ventricle beats as they both appear on the IC signals (by looking at body surface signals in parallel). Such a differentiation may provide better input for the algorithms disclosed herein, as patterns may be more aligned with incoming beats. Further, by applying advanced reference annotations, when determining a template, the system may automatically select a beat which is not a fused activation of atrium and ventricle, which may result in a template of superior quality.

When the advanced reference annotation is used, the time difference for a WOI is calculated between the last reference annotation in the saved window and its nearest ventricle annotation. If the time difference is equal or below a threshold time (e.g., 100 mSec), the pattern within the WOI may be defined as an overlapping activation pattern. In such case, the POI is calculated around the previous reference annotation, second to last in the saved WOI.

According to an exemplary embodiment of the present invention, if an advanced reference annotation is not used, the POI is determined based on the last annotation in the saved WOI.

According to an exemplary embodiment of the present invention, the POI may be automatically determined based on the pattern exhibited by the signals within a WOI. The POI may be set around the last reference annotation in the saved template window and may be edited by the user. A POI may be calculated based on an activity segment defined around the reference annotations provided by a medical professional 22 or automatically provided, the reference annotation defining the WOI. An activity segment may be defined as set forth in Equation 1 set forth below:

$$Activity\ Segment = [Annotation\ TS - winSize\ ,\ Annotation\ TS\ + winSize]$$

wherein, for example:

$$winSize = \begin{cases} 150\ mSec & for\ ECG\ signals \\ 100\ mSec & for\ EGM\ signals \end{cases}$$

In the definition of an Activity Segment above, the TS represents a time stamp. It will be noted that the automated POI selection technique disclosed herein may be applied to the IC EGM signals disclosed herein as well as to body surface pattern matching with signals provided by body surface electrodes.

An activity signal may be calculated for the signals defined by the activity segment by applying a median filter with a window of a given time (e.g., 15 milliseconds). The activity signal may be calculated as set forth in Equation 2 below:

$$Activity\ Signal = MedianFilter \left( \sum \left| \frac{dv}{dt}\ Activity\ Segment \right| \right)$$

An activity threshold may be calculated based on the activity signal by applying Equation 3 below:

$$Activity\ Threshold = 0.4 * \big( max(Activity\ Signal) - min(Activity\ Signal) \big)$$

A first and last intersection of the activity signal with the activity threshold may be set as the first intersection and the last intersection such that the POI is determined based on Equation 4 as set forth below:

$$POI = [First\ Intersection\ TS - 25mSec, Last\ Intersection\ TS + 25mSec]$$

As disclosed herein, the template POI may be based on the WOI as identified by a medical professional 22 or as automatically determined.

At step 230, the multiple channels corresponding to the IC EGM signals provided by respective multiple electrodes 36 of the catheter 29 may be weighted based on the template's signal derivate such that prominent activations within the multiple channel IC EGM signals sensed by the electrodes 36 are emphasized and other activations are deemphasized. In other words, correlation is first calculated for every channel among the multiple channels. Then, a final correlation is calculated for all channels. Each channel contributes differently, such that the sharpest channels have the most impact on the final correlation. Flat channels will have almost no effect upon the final correlation. The subsequent electrical activity may be unipolar IC EGM signals that are captured by the electrodes of the catheter 29.

At step 230 of the process 200 of FIG. 2, the correlation function may utilize a weighting mechanism to compare each annotated incoming beat of the subsequent electrical activity against the pre-defined POI of the template. The correlation function may be defined as set forth in Equation 5 below:

$$SignalCorr_i = \frac{TemplateSignal_i \cdot CandidateSignal_i}{\sqrt{TemplateSignal_i \cdot TemplateSignal_i} * NormSignal}$$

wherein:

$$NormSignal = \begin{cases} \sqrt{TemplateSig_i \cdot TemplateSig_i} & if\ 0.1 \times TemplateSig_i \cdot TemplateSig_i > CandidateSig_i \cdot CandidateSig_i \\ \sqrt{CandidateSig_i \cdot CandidateSig_i} & otherwise \end{cases}$$

Weights may be applied to each of the channels that correspond to the respective unipolar IC EGM signals received from each electrode of a catheter. The channel weights are calculated based on the maximum slope of the template's signals, indicating the predominant channels which should have more effect on the final correlation outcome. Notably, the maximum slope of the template signals is the derivative function of the template signals such that the channel weights are derivate of the template slopes. By using the derivative based function, sharp activations may be distinguishable from shallow activations which may provide a better template match as compared to an amplitude-based function which may provide more inconclusive results for certain activations. For example, the channel weights of each of the channels may be calculated based on Equation 6 set forth below:

$$W_i = \frac{maxSlope_i}{\sum maxSlope_i}$$

wherein:

$$maxSlope_i = Min(maxThreshold, \quad Max(differences(SignalCorr_i)))$$

The maxThreshold may be defined as a predefined value (e.g., .2).

At step 240 of the process 200 of FIG. 2, each annotated beat in the subsequent electrical activity may be compared against the template pattern. Step 240 includes at least one or more of the steps 240a through 240c, as further disclosed herein. Annotation is a marking on the ECG/EGM signals, where an activation occurs. That activation can then be correlated with the selected pattern.

At step 240a, a preprocessing step may be applied such that input unipolar IC EGM signals are sampled at a sample rate (e.g., 1 Khz) after passing through a FIR low pass filter ("LPF") filter (e.g., a 250Hz FIR LPF filter). This additional filtering may be applied to remove the baseline wander, caused by patient movement and respiration.

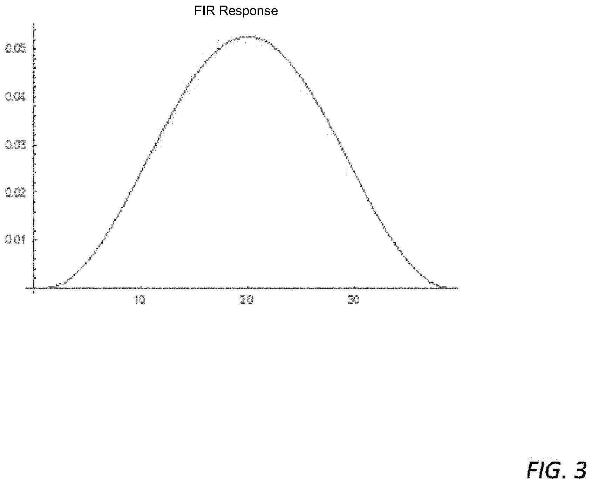

During the pre-processing step 240a, a median filter may be applied to the input IC EGM signals with a given size (e.g., a size of +/- 20 milliseconds). To smoothen the signal and remove artifacts, an additional FIR filter may be applied on the median filtered signal, padded with a number of zero samples (e.g., 20 zero samples). FIG. 3 shows an example FIR filter setting forth the frequencies that are interesting and desired and removing the frequencies that are not interesting and being discarded.

The FIR filter coefficients may be calculated by Equation 7 set forth below:

$$FIRcoeff = \frac{0.5 * (1 - Cos[2 * \pi * x])}{\sum 0.5 * (1 - Cos[2 * \pi * x])}\ for\ x \in \{0,1\ with\ steps\ of\ \frac{1}{38}\}$$

As part of the preprocessing step, the median filtered signal may be subtracted from the original signal and may remove the baseline wander while preserving the input IC EGM signal morphology. The subtraction may be represented by the following Equation 8 set forth below:

$$FilteredSignal = Signal - FIR(MedianFilteredSignal)$$

At step 240b, a correlation may be calculated between the template and a current activation of each channel such that an overall correlation value is determined based on the weights of each channel.

A single correlation value for all the channels may be determined. The integrative correlation value may be calculated by the following Equation 9 set forth below:

$$Corr = \sum W_i * SignalCorr_i$$

Notably, the signal correlation for all channels is considered when determining a correlation between the template and subsequent electrical activity.

A correlation value may be determined for each beat of the subsequent electrical activity such that for each incoming annotated beat, the correlation value is applied over a window of +/- T (e.g., 40 milliseconds) around the beat's reference annotation such that a correlation value is determined for each sampling point on the heart. The maximum correlation value in a given segment is selected as the beat's correlation value with the defined template.

According to an exemplary embodiment of the present invention, the correlation values may only be determined for a subset of channels such that not all the available IC EGM channels contribute to the correlation value. For example, where the left and right atria are dissociated, i.e., not synchronized in their contractions as they should be, or when one or more channels exhibit greater than a threshold amount of noise, such channels may be excluded from determining the correlation value. The exclusion of such channels may be automatic (e.g., channels that exhibit greater than a threshold amount of noise may be automatically excluded) or may be user defined.

At step 240c, a moving window defined by the template POI may be used to determine correlation values for each shift of the moving window. In other words, the template is laid over IC ECM signals of subsequent electrical activity and the template is shifted from side to side over the subsequent electrical activity to maximize the correlation between the two. A final score for each shift of the moving window may be selected as the maximum correlation value determined for the subsequent electrical activity beat.

During implementation of the phase shift, the processor 40 may iteratively change the phase of the IC EGM signal for a given beat, relative to the phase of the template POI. The processor 40 may use the value of the overall correlation from Equation 9 as well as the correlation threshold (e.g., as provided by a user which may be, for example, .9) as an input for the phase shift implementation.

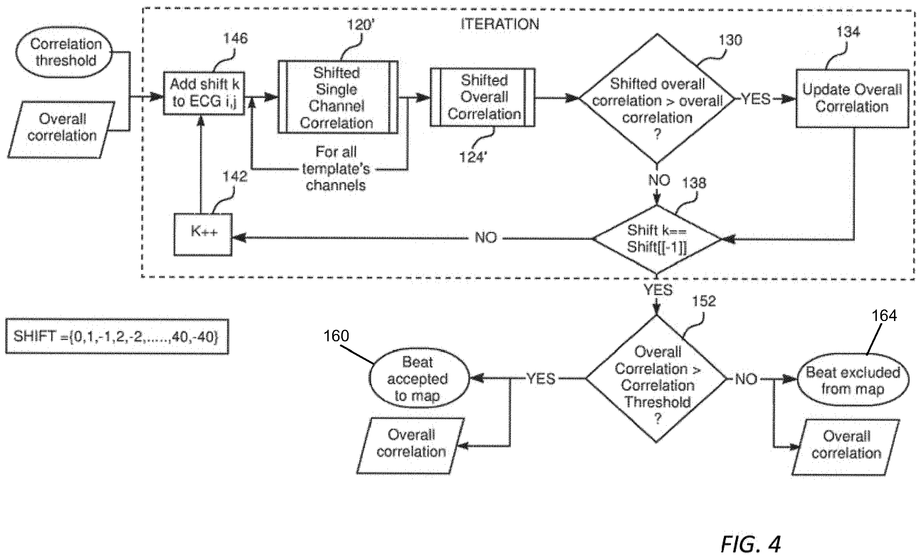

FIG. 4 shows, in an iteration set of blocks, the iterative process performed by a shifted single channel correlation block 120', shifted overall correlation block 124', and phase shift block 142. As shown in FIG. 4, at each iteration, the processor repeats the first two steps, in a "shifted single channel correlation" block 120' and a "shifted overall correlation" block 124'. The process starts with a correlation for every single channel and then calculates the total correlation with weights added. During the iterations, the processor 40 (Fig. 1) determines a maximum value of the overall correlation as described herein.

In the example shown in FIG. 4, the phase shift iterations are performed every 1 msec in a $\pm 40$ msec time frame measured from the annotation of the beat being analyzed. An index k defines the phase shift being evaluated, and k={ -40, ... 0, ... +40}. At each iteration, the value of the shifted overall correlation is compared to a previous maximum correlation in a comparison block 130, and if the comparison block 130 returns a positive value, then the overall correlation is updated in an update block 134.

Subsequent to a return (e.g., "YES" (positive) or "NO" (negative)), control continues to the comparison block 138, which checks if there are any more values of index k to be iterated. If there are, k is incremented in an incremental block 142, the new value of k is applied to the ECG signal in a signal block 146, and the flowchart returns to block 120'.

If the iterations have completed, then control continues to a final comparison block indicated at 152 of Fig. 4. At block 152, the updated overall correlation value obtained from block 134, which is a maximum value of correlation for the subsequent electrical activity obtained through the iterative process as described above, is compared with a predetermined threshold correlation value. Typically, a correlation threshold may be set at a high threshold, e.g., 0.90. Alternatively, the correlation threshold may be set somewhat lower, e.g., 0.80.

As set forth in block 152 of Fig. 4, if the maximum correlation value for the subsequent electrical activity is greater than the threshold value, then the subsequent electrical activity is assumed to represent the same arrhythmia as the morphology pattern identified in the POI template pattern. In this case, the processor 40 may add this subsequent electrical activity to the map of the heart (82 in Fig. 1) as indicated at 160. Subsequent electrical signals that are accepted to the map of the heart (82 in Fig. 1) are collected and displayed on the map of the heart 82 (Fig. 1) on the display 80 (Fig. 1) as problematic electrical activity. Alternatively, as set forth at block 152, if the maximum correlation value of the subsequent electrical activity is less than the threshold value, then the subsequent electrical activity signal is assumed to represent a different arrhythmia from the morphology pattern identified in the POI template pattern, or is assumed to represent a sinus rhythm, an ectopic beat, or a mechanically induced beat, and the subsequent electrical activity signal is excluded from the map as indicated at 164.

According to an exemplary embodiment, subsequent electrical activity may be compared to a template by creating a sequence of activation comparisons, by annotating each channel's activation and measuring the time difference between all channels as a word of value compared between each beat, and an RMS (root mean square) difference can indicate of sequence change. Root mean square is used for error calculation in known ways.

Alternatively, for each channel, a POI template pattern is placed over subsequent electrical activity, i.e., a subsequent heartbeat, at an initial position and a correlation value is calculated. Next, weighting for all channels at this initial position is performed and a final correlation value for all channels is calculated. Next, the POI template pattern is phase shifted, or moved, from the initial position to a second position over the same subsequent beat. For example, the phase shift may be 1 msec to the left from the initial position. A correlation value is then calculated for each channel for this new position of the POI template pattern over the subsequent beat. Weighting for all channels is performed and a final correlation for all channels for this second position is calculated. Next, the POI template pattern is phase shifted over the subsequent beat from the second position to a third position and the process is repeated until the POI template pattern has been placed over the subsequent beat at all positions within a range of positions and a final correlation for all channels has been obtained for each position within the range of positions. For example, the POI template pattern may be shifted to different positions within a range of positions over the subsequent beat, e.g., within a ±40 msec time frame, and in increments of 1msec for each different position. The position having the highest correlation is the one that is selected.

At step 250 of the process 200 of FIG. 2, if the maximum correlation value for a given beat of the subsequent electrical activity is greater than a threshold correlation value (e.g., 0.8 or .9), then the given beat of the subsequent electrical activity may be determined to be correlated to the template. According to an embodiment, the threshold correlation may be provided by a user (e.g., medical professional) and/or may have a default value (e.g., 0.8 or .9) which may be applied absent a change to the threshold correlation.

The overall correlation coefficient calculated by Equation 9 depends on the phase of the ECG signal being tested relative to the phase of the morphology pattern.

Any of the functions and methods described herein can be implemented in a general-purpose computer, a processor, or a processor core. Suitable processors include, by way of example, a general- purpose processor, a special purpose processor, a conventional processor, a digital signal processor (DSP), a plurality of microprocessors, one or more microprocessors in association with a DSP core, a controller, a microcontroller, Application Specific Integrated Circuits (ASICs), Field Programmable Gate Arrays (FPGAs) circuits, any other type of integrated circuit (IC), and/or a state machine. Such processors can be manufactured by configuring a manufacturing process using the results of processed hardware description language (HDL) instructions and other intermediary data including netlists (such instructions capable of being stored on a computer-readable media). The results of such processing can be mask works that are then used in a semiconductor manufacturing process to manufacture a processor which implements features of the disclosure.

Any of the functions and methods described herein can be implemented in a computer program, software, or firmware incorporated in a non-transitory computer-readable storage medium for execution by a general-purpose computer or a processor. Examples of non-transitory computer-readable storage mediums may include read only memory (ROM), random access memory (RAM), a register, cache memory, semiconductor memory devices, magnetic media such as internal hard disks and removable disks, magneto-optical media, and optical media such as CD-ROM disks, and digital versatile disks (DVDs).

It should be understood that many variations are possible based on the disclosure herein. Although features and elements are described above in particular combinations, each feature or element can be used alone without the other features and elements or in various combinations with or without other features and elements.

**Claims**

1. A computer implemented method for intra-cardiac pattern matching, the method comprising:

   receiving unipolar pattern intra-cardiac (IC) electrogram (EGM) signals for an area of a heart, from a plurality of activity channels corresponding to a plurality of electrodes of a catheter;
   selecting a window of interest (WOI) of the IC EGM signals, the WOI representing the entire cycle length for a single heartbeat;
   selecting a pattern of interest (POI), the POI encompassing at least a portion of the WOI, and corresponding to a specific portion of the WOI where an arrhythmia activation is exhibited;
   generating a template POI representative of the arrhythmia activation, the template POI formed of a plurality of template channels and based upon the IC EGM signals within the WOI;
   receiving subsequent electrical activity comprising IC EGM signals from the plurality of activity channels and applying weights to the plurality of activity channels to generate weighted subsequent electrical activity;
   generating a correlation score by comparing the weighted subsequent electrical activity to the template POI; and
   determining that the correlation score is above a threshold correlation score;
   **characterized in that** the weights are based on a derivative of a pattern within each template channel.

2. The computer implemented method of claim 1, further comprising providing a visual indication corresponding to the area of the heart based on determining that the correlation score is above the threshold correlation score.

3. The computer implemented method of claim 2, wherein the visual indication is at least one of a color, a pattern, and a marking.

4. The computer implemented method of claim 1, wherein the POI is received based on user provided reference annotations.

5. The computer implemented method of claim 1, further comprising filtering the subsequent electrical activity using at least one of a median filter and a finite impulse response (FIR) filter.

6. The computer implemented method of claim 5, wherein filtering the subsequent electrical activity removes baseline wander.

7. The computer implemented method of claim 1, wherein the correlation score is a cumulative value for the weighted subsequent electrical activity.

8. The computer implemented method of claim 1, further comprising defining a moving window based on the POI.

9. The computer implemented method of claim 8, further comprising determining a plurality of correlation scores based on each shift of the moving window.

10. The computer implemented method of claim 9, wherein the correlation score is based on a maximum correlation score.

11. The computer implemented method of claim 1, wherein the correlation score is determine based on a subset of the subsequent electrical activity.

12. The computer implemented method of claim 11, wherein the subset of the subsequent electrical activity is determined based on at least one of an atria dissociation and a noise threshold.

13. An apparatus for identifying cardiac arrhythmia activations by matching subsequent electrical activity with a template created based upon an identified cardiac arrhythmia activation, said apparatus comprising:

one or more catheters with respective one or more electrodes arranged for insertion into the heart of a human patient for acquiring unipolar pattern intra-cardiac (IC) electrogram (EGM) signals from an area of the heart; and, a processor arranged for receiving the IC EGM signals from the one or more catheters, the processor arranged for receiving a pattern of interest (POI) of the IC EGM signals, the POI comprising a portion of the IC EGM signals corresponding to the cardiac arrhythmia activation; generating a template pattern of interest (POI) representative of the arrhythmia activation, the template POI comprising a plurality of template channels based on the IC EGM signals within the WOI; receiving a subsequent electrical activity comprising IC EGM signals from the plurality of activity channels and applying weights to the plurality of activity channels to generate weighted subsequent electrical activity; generating a correlation score by comparing the weighted subsequent electrical activity to the template POI; and determining that the correlation score is above a threshold correlation score; and **characterized in that** the weights are based on a derivative of a pattern within each template channel.

14. The apparatus of claim 13, additionally comprising at least one filter to remove respiratory or other movement.

15. The apparatus of claim 14, additionally comprising a display screen arranged for displaying subsequent electrical activity having a correlation score exceeding a threshold amount to indicate the arrhythmia activation.

16. The apparatus of claim 15, wherein the subsequent electrical activity having a correlation score exceeding a threshold amount is incorporated into a map of the heart appearing on the display screen, wherein areas of the heart exhibiting the arrhythmia activation are emphasized.

17. The apparatus of claim 13 wherein the catheter comprises a probe disposed within a sheath.

18. The apparatus of claim 17, wherein the catheter comprises at least one of a lasso catheter and a shaft-like catheter.

19. The apparatus of claim 13, wherein the processor additionally comprises at least one filter arranged to be applied to the IC EGM signals corresponding to a cardiac condition prior to generation of the template POI.

20. The apparatus of claim 13, wherein the processor additionally comprises at least one of a median filter and a finite impulse response (FIR) filter for filtering subsequent electrical activity.

21. The apparatus of claim 13, additionally comprising a memory accessible by the processor, wherein the template POI may be stored in the memory.

22. The apparatus of claim 13, wherein the processor is located within an operating console.

23. The apparatus of claim 13, wherein the processor further comprises a central processing unit including noise reduction circuitry.

24. The apparatus of claim 13, additionally comprising a magnetic tracking system for tracking the one or more catheters when inserted into the heart of a human patient.

**Patentansprüche**

1. Computerimplementiertes Verfahren zum intrakardialen Musterabgleich, wobei das Verfahren umfasst:

Empfangen von intrakardialen Elektrogrammsignalen (Intra-Cardiac Electrogram, IC EGM) mit unipolarem Muster für einen Bereich eines Herzens, über mehrere Aktivitätskanäle, die mehreren Elektroden eines Katheters entsprechen;
Auswählen eines Fensters von Interesse (Window of Interest, WOI) der IC-EGM-Signale, wobei das WOI die gesamte Zykluslänge für einen einzelnen Herzschlag darstellt;
Auswählen eines Musters von Interesse (Pattern of Interest, POI), wobei das POI wenigstens einen Abschnitt des WOI umfasst und einem spezifischen Abschnitt des WOI entspricht, wo eine Arrhythmieaktivierung vorliegt;
Generieren einer POI-Vorlage, die für die Arrhythmieaktivierung steht, wobei die POI-Vorlage aus mehreren Vorlagekanälen gebildet ist und auf den IC-EGM-Signalen innerhalb des WOI basiert;
Empfangen einer nachfolgenden elektrischen Aktivität, umfassend IC-EGM-Signale von den mehreren Aktivitätskanälen, und Anwenden von Gewichtungen auf die mehreren Aktivitätskanäle, um eine gewichtete nachfolgende elektrische Aktivität zu generieren;
Generieren einer Korrelationsbewertung durch Vergleichen der gewichteten nachfolgenden elektrischen Aktivität mit der POI-Vorlage; und
Bestimmen, dass die Korrelationsbewertung über einer Schwellwert-Korrelationsbewertung liegt;
**dadurch gekennzeichnet, dass** die Gewichtungen auf einer Ableitung eines Musters innerhalb jedes Vorlagenkanals basieren.

2. Computerimplementiertes Verfahren nach Anspruch 1, ferner umfassend das Bereitstellen einer visuellen Angabe, die dem Bereich des Herzens entspricht, basierend auf dem Bestimmen, dass die Korrelationsbewertung über der Schwellwert-Korrelationsbewertung liegt.

3. Computerimplementiertes Verfahren nach Anspruch 2, wobei die visuelle Angabe wenigstens eines von einer Farbe, einem Muster und einer Markierung ist.

4. Computerimplementiertes Verfahren nach Anspruch 1, wobei das POI basierend auf benutzerseitig bereitgestellten Referenzanmerkungen empfangen wird.

5. Computerimplementiertes Verfahren nach Anspruch 1, ferner umfassend das Filtern der nachfolgenden elektrischen Aktivität unter Verwendung von wenigstens einem von einem Medianfilter und einem Filter mit finiter Impulsantwort (Finite Impulse Response, FIR).

6. Computerimplementiertes Verfahren nach Anspruch 5, wobei das Filtern der nachfolgenden elektrischen Aktivität ein Grundlinienwandern entfernt.

7. Computerimplementiertes Verfahren nach Anspruch 1, wobei die Korrelationsbewertung ein kumulativer Wert für die gewichtete nachfolgende elektrische Aktivität ist.

8. Computerimplementiertes Verfahren nach Anspruch 1, ferner umfassend das Definieren eines beweglichen Fensters basierend auf dem POI.

9. Computerimplementiertes Verfahren nach Anspruch 8, ferner umfassend das Bestimmen mehrerer Korrelationsbewertungen basierend auf jeder Verschiebung des beweglichen Fensters.

10. Computerimplementiertes Verfahren nach Anspruch 9, wobei die Korrelationsbewertung auf einer maximalen Korrelationsbewertung basiert.

11. Computerimplementiertes Verfahren nach Anspruch 1, wobei die Korrelationsbewertung basierend auf einer Teilmenge der nachfolgenden elektrischen Aktivität bestimmt wird.

12. Computerimplementiertes Verfahren nach Anspruch 11, wobei die Teilmenge der nachfolgenden elektrischen Aktivität basierend auf wenigstens einem von einer Vorhofdissoziation und einem Rauschschwellwert bestimmt wird.

13. Einrichtung zum Identifizieren kardialer Arrhythmieaktivierungen durch Abgleichen einer nachfolgenden elektrischen Aktivität mit einer Vorlage, die basierend auf einer identifizierten kardialen Arrhythmieaktivierung erstellt wird, wobei die Einrichtung umfasst:

einen oder mehrere Katheter mit jeweils einer oder mehreren Elektroden, ausgelegt zum Einführen in das Herz eines menschlichen Patienten, um intrakardiale Elektrogrammsignale (Intra-Cardiac Electrogram, IC EGM) mit unipolarem Muster aus einem Bereich des Herzens zu beziehen; und, einen Prozessor, ausgelegt zum Empfangen der IC-EGM-Signale von den ein oder mehreren Kathetern, wobei der Prozessor ausgelegt ist zum:

Empfangen eines Musters von Interesse (Pattern of Interest, POI) der IC-EGM-Signale, wobei das POI einen Abschnitt der IC-EGM-Signale umfasst, welcher der kardialen Arrhythmieaktivierung entspricht; Generieren einer POI-Vorlage (Pattern of Interest, Muster von Interesse), die für die Arrhythmieaktivierung steht, wobei die POI-Vorlage mehrere Vorlagenkanäle umfasst, basierend auf den IC-EGM-Signalen innerhalb des WOI; Empfangen einer nachfolgenden elektrischen Aktivität, umfassend IC-EGM-Signale von den mehreren Aktivitätskanälen, und Anwenden von Gewichtungen auf die mehreren Aktivitätskanäle, um eine gewichtete nachfolgende elektrische Aktivität zu generieren; Generieren einer Korrelationsbewertung durch Vergleichen der gewichteten nachfolgenden elektrischen Aktivität mit der POI-Vorlage; und Bestimmen, dass die Korrelationsbewertung über einer Schwellwert-Korrelationsbewertung liegt; und **dadurch gekennzeichnet, dass** die Gewichtungen auf einer Ableitung eines Musters innerhalb jedes Vorlagenkanals basieren.

14. Einrichtung nach Anspruch 13, zusätzlich umfassend wenigstens ein Filter zum Entfernen von Atembewegungen oder sonstigen Bewegungen.

15. Einrichtung nach Anspruch 14, zusätzlich umfassend einen Anzeigeschirm, ausgelegt zum Anzeigen nachfolgender elektrischer Aktivitäten mit einer Korrelationsbewertung, die einen Schwellwert überschreiten, um die Arrhythmieaktivierung anzugeben.

16. Einrichtung nach Anspruch 15, wobei die nachfolgende elektrische Aktivität mit einer Korrelationsbewertung, die einen Schwellwert überschreitet, in eine Karte des Herzens integriert ist, die auf dem Anzeigeschirm erscheint, wobei Bereiche des Herzens, in denen die Arrhythmieaktivierung vorliegt, hervorgehoben sind.

17. Einrichtung nach Anspruch 13, wobei der Katheter eine Sonde umfasst, die innerhalb einer Hülle angeordnet ist.

18. Einrichtung nach Anspruch 17, wobei der Katheter wenigstens eines von einem Lassokatheter und einem schaftartigen Katheter ist.

**19.** Einrichtung nach Anspruch 13, wobei der Prozessor zusätzlich wenigstens ein Filter umfasst, das dafür ausgelegt ist, vor Generierung der POI-Vorlage auf die IC-EGM-Signale angewendet zu werden, welche einem kardialen Zustand entsprechen.

**20.** Einrichtung nach Anspruch 13, wobei der Prozessor zusätzlich wenigstens eines von einem Medianfilter und einem Filter mit finiter Impulsantwort (Finite Impulse Response, FIR) zum Filtern nachfolgender elektrischer Aktivitäten umfasst.

**21.** Einrichtung nach Anspruch 13, zusätzlich umfassend einen Speicher, der für den Prozessor zugänglich ist, wobei die POI-Vorlage in dem Speicher gespeichert sein kann.

**22.** Einrichtung nach Anspruch 13, wobei sich der Prozessor innerhalb einer Betriebskonsole befindet.

**23.** Einrichtung nach Anspruch 13, wobei der Prozessor ferner eine zentrale Verarbeitungseinheit umfasst, die eine Rauschreduzierungsschaltung aufweist.

**24.** Einrichtung nach Anspruch 13, zusätzlich umfassend ein magnetisches Verfolgungssystem zum Verfolgen der ein oder mehreren Katheter, wenn diese in das Herz eines menschlichen Patienten eingeführt werden.

**Revendications**

**1.** Procédé mis en œuvre par ordinateur pour la mise en correspondance de modèles intracardiaques, le procédé comprenant :

la réception de signaux d'électrogramme (EGM) intracardiaque (IC) de modèle unipolaire pour une zone d'un coeur, en provenance d'une pluralité de canaux d'activité correspondant à une pluralité d'électrodes d'un cathéter ;
la sélection d'une fenêtre d'intérêt (WOI) des signaux EGM IC, la WOI représentant la longueur totale du cycle pour un seul battement de coeur ;
la sélection d'un modèle d'intérêt (POI), le POI englobant au moins une partie de la WOI, et correspondant à une partie spécifique de la WOI où se manifeste une activation d'arythmie ;
la génération d'un POI de gabarit représentatif de l'activation d'arythmie, le POI de gabarit étant formé d'une pluralité de canaux de gabarit et basé sur les signaux EGM IC à l'intérieur de la WOI ;
la réception d'une activité électrique ultérieure comprenant des signaux EGM IC en provenance de la pluralité de canaux d'activité et l'application de pondérations à la pluralité de canaux d'activité pour générer une activité électrique ultérieure pondérée ;
la génération d'un score de corrélation en comparant l'activité électrique ultérieure pondérée au POI de gabarit ; et
la détermination établissant si le score de corrélation est supérieur à un score de corrélation seuil ;
**caractérisé en ce que** les pondérations sont basées sur une dérivée d'un modèle au sein de chaque canal de gabarit.

**2.** Procédé mis en œuvre par ordinateur selon la revendication 1, comprenant en outre la fourniture d'une indication visuelle correspondant à la zone du coeur sur la base de la détermination établissant que le score de corrélation est supérieur au score de corrélation seuil.

**3.** Procédé mis en œuvre par ordinateur selon la revendication 2, dans lequel l'indication visuelle est au moins l'un parmi une couleur, un modèle et un marquage.

**4.** Procédé mis en œuvre par ordinateur selon la revendication 1, dans lequel le POI est reçu sur la base d'annotations de référence fournies par un utilisateur.

**5.** Procédé mis en œuvre par ordinateur selon la revendication 1, comprenant en outre le filtrage de l'activité électrique ultérieure en utilisant au moins l'un parmi un filtre médian et un filtre à réponse impulsionnelle finie (RIF).

**6.** Procédé mis en œuvre par ordinateur selon la revendication 5, dans lequel le filtrage de l'activité électrique ultérieure supprime l'ondulation de la ligne de base.

**7.** Procédé mis en œuvre par ordinateur selon la revendication 1, dans lequel le score de corrélation est une valeur cumulée pour l'activité électrique ultérieure pondérée.

**8.** Procédé mis en œuvre par ordinateur selon la revendication 1, comprenant en outre la définition d'une fenêtre mobile basée sur le POI.

**9.** Procédé mis en œuvre par ordinateur selon la revendication 8, comprenant en outre la détermination d'une pluralité de scores de corrélation sur la base de chaque décalage de la fenêtre mobile.

**10.** Procédé mis en œuvre par ordinateur selon la revendication 9, dans lequel le score de corrélation est basé sur un score de corrélation maximal.

**11.** Procédé mis en œuvre par ordinateur selon la revendication 1, dans lequel le score de corrélation est déterminé sur la base d'un sous-ensemble de l'activité électrique ultérieure.

**12.** Procédé implémenté par ordinateur selon la revendication 11, dans lequel le sous-ensemble de l'activité électrique ultérieure est déterminé sur la base d'au moins l'un parmi un seuil de dissociation des oreillettes et un seuil de bruit.

**13.** Appareil permettant d'identifier des activations d'arythmie cardiaque en faisant correspondre une activité électrique ultérieure avec un gabarit créé sur la base d'une activation d'arythmie cardiaque identifiée, ledit appareil comprenant :

un ou plusieurs cathéters dotés d'une ou de plusieurs électrodes respectives conçues pour être insérées dans le coeur d'un patient humain afin d'acquérir des signaux d'électrogramme (EGM) intracardiaque (IC) à modèle unipolaire provenant d'une zone du coeur ; et,
un processeur conçu pour recevoir les signaux EGM IC en provenance des un ou plusieurs cathéters, le processeur étant conçu pour
recevoir un modèle d'intérêt (POI) des signaux EGM IC, le POI comprenant une partie des signaux EGM IC correspondant à l'activation d'arythmie cardiaque ;
générer un modèle d'intérêt (POI) de gabarit représentatif de l'activation d'arythmie, le POI de gabarit comprenant une pluralité de canaux de gabarit basés sur les signaux EGM IC à l'intérieur de la WOI ;
recevoir une activité électrique ultérieure comprenant des signaux EGM IC en provenance de la pluralité de canaux d'activité et appliquer des pondérations à la pluralité de canaux d'activité pour générer une activité électrique ultérieure pondérée ;
générer un score de corrélation en comparant l'activité électrique ultérieure pondérée au POI de gabarit ; et
déterminer que le score de corrélation est supérieur à un score de corrélation seuil ; et
**caractérisé en ce que** les pondérations sont basées sur une dérivée d'un modèle dans chaque canal de gabarit.

**14.** Appareil selon la revendication 13, comprenant en outre au moins un filtre pour éliminer les mouvements respiratoires ou autres.

**15.** Appareil selon la revendication 14, comprenant en outre un écran d'affichage conçu pour afficher une activité électrique ultérieure ayant un score de corrélation dépassant une quantité seuil pour indiquer l'activation d'arythmie.

**16.** Appareil selon la revendication 15, dans lequel l'activité électrique ultérieure ayant un score de corrélation dépassant une quantité seuil est incorporée dans une carte du coeur apparaissant sur l'écran d'affichage, dans laquelle des zones du coeur présentant l'activation d'arythmie sont mises en évidence.

**17.** Appareil selon la revendication 13 dans lequel le cathéter comprend une sonde disposée dans une gaine.

**18.** Appareil selon la revendication 17, dans lequel le cathéter comprend au moins un cathéter lasso et un cathéter en forme de tige.

**19.** Appareil selon la revendication 13, dans lequel le processeur comprend en outre au moins un filtre conçu pour être appliqué aux signaux EGM IC correspondant à un état cardiaque avant la génération du POI de gabarit.

**20.** Appareil selon la revendication 13, dans lequel le processeur comprend en outre au moins l'un parmi un filtre médian et un filtre à réponse impulsionnelle finie (RIF) pour filtrer l'activité électrique ultérieure.

**21.** Appareil selon la revendication 13, comprenant en outre une mémoire accessible par le processeur, dans lequel le POI de gabarit peut être stocké dans la mémoire.

**22.** Appareil selon la revendication 13, dans lequel le processeur est situé à l'intérieur d'une console d'exploitation.

**23.** Appareil selon la revendication 13, dans lequel le processeur comprend en outre une unité centrale de traitement comprenant des circuits de réduction du bruit.

**24.** Appareil selon la revendication 13, comprenant en outre un système de suivi magnétique pour suivre les un ou plusieurs cathéters lorsqu'ils sont insérés dans le coeur d'un patient humain.

FIG. 1

FIG. 2

EP 3 831 304 B1

FIG. 3

FIG. 4

EP 3 831 304 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20180008203 A1
- US 20170042443 A1
- US 20180042510 A1
- US 20180199847 A1
- US 9259165 B, Rubenstein